# EUROPEAN PATENT SPECIFICATION

(11) **EP 4 355 211 B1**
(45) Date of publication and mention of the grant of the patent: **16.09.2026**
(21) Application number: 22748177.7
(22) Date of filing: 05.07.2022
(51) Int. Cl.: A61B 5/0538

(54) **IMPEDANCE MONITORING VASCULAR ACCESS DEVICE**
IMPEDANZÜBERWACHENDE GEFÄSSZUGANGSVORRICHTUNG
DISPOSITIF D'ACCÈS VASCULAIRE DE SURVEILLANCE D'IMPÉDANCE

(30) Priority: 06.07.2021 US 202163218824 P
(43) Date of publication of application: 24.04.2024
(73) Proprietor: Bard Access Systems, Inc., Salt Lake City, UT 84116 (US)
(72) Inventor: SOWARDS, Steffan, Salt Lake City, UT 84124 (US); MISENER, Anthony, K., Bountiful, UT 84010 (US); URRY, Robin, Scott, Syracuse, UT 84075 (US); MCLAUGHLIN, William, Robert, Bountiful, UT 84010 (US); MESSERLY, Shayne, Kaysville, UT 84037 (US)
(74) Representative: Hoffmann Eitle
(86) International application number: PCT/US2022/036142
(87) International publication number: WO 2023/283199

(56) References cited:
- US-A1- 2014 180 278
- US-A1- 2015 272 451
- US-A1- 2016 067 464
- US-A1- 2017 347 896

## Description

### PRIORITY

This application claims the benefit of priority to U.S. Provisional Application No. 63/218,824, filed July 6, 2021.

### BACKGROUND

Medical devices such as catheters are employed to treat patients in several ways including delivering fluids to a patient and draining fluids from a patient. Catheter treatments may extend over long periods, such as several days or more. It is typical for complications to arise during a medical process requiring corrective action before the medical process may continue. Complications may interrupt the medical process and, in some instances, the interruption may go undetected for extended periods. Complications often include the loss of patency of the catheter and corrective actions often include process to restore patency while the catheter is inserted into the patient. In some cases, the complications may cause the catheter to be replaced which may expose the patient to significant risk. While medical processes are very common, the processes are not risk free. Insertion and proper placement of the catheter often require intense focus on the part of the clinician in combination with visualizing the location of the catheter using imaging equipment. In short, patient risk and healthcare expense associated with medical processes can be reduced with simple devices and methods for the insertion of catheters and the subsequent monitoring of the medical process. US 2015/0272451 A1 discloses an introducer including a sheath. A plurality of electrodes are on the sheath. An impedance assessment unit is provided to compute an impedance between a pair of electrodes. US 2016 067464 A1 discloses a self-monitoring catheter that measures changes in impedance between two electrodes in the catheter. US 2017 347896 A1 discloses a system for assessing the occlusion of a region to blood flow by measuring impedance between two electrodes where one of the electrodes is upstream of an inflatable member and the other of the electrodes is downstream of the inflatable member. US 2014 180278 A1 discloses a catheter configured to determine a respiratory rate based on an impedance value measured by the catheter.

The systems and methods described herein may aid in the insertion of medical devices, including catheters, and the monitoring of a medical process performed during and/or after insertion.

### SUMMARY OF THE INVENTION

Briefly summarized, disclosed herein is a system for monitoring a medical process, including a medical device (e.g., a catheter, a needle, a stylet, a guidewire, an introducer or a combination thereof) configured for insertion within a vasculature of a patient, a plurality of electrodes coupled with the catheter, and a monitoring module electrically coupled with the plurality of electrodes, the module including logic stored in memory that, when executed by one or more processors, causes performance of operations. The operations include (i) transmitting an electrical signal between a first electrode and a second electrode, (ii) determining an electrical impedance between the first electrode and the second electrode, (iii) correlating one or more impedance measurements with an infiltration of an infusate, and (iv) providing a notification to the operator when the determined electrical impedance is outside a predefined impedance range.

In some embodiments, the operations include (i) transmitting electrical signals between a plurality of first electrodes and a plurality of second electrodes, (ii) determining electrical impedances between a plurality of electrode pairs, and (iii) providing a notification to the operator when one or more determined electrical impedances is outside one or more predefined impedance ranges.

The electrodes may be disposed along a monitoring length of the elongate member, and at least a subset of the plurality of electrodes may be disposed within a lumen of the medical device.

The operations may include correlating one or more impedance measurements with an occlusion of the lumen, and the occlusion may be one of a thrombotic occlusion, a chemical occlusion, or a mechanical occlusion. The operations may further include correlating one or more impedance measurements with a magnitude of the occlusion and/or a location of the occlusion along the medical device.

In some embodiments, the operations further include correlating one or more impedance measurements with an air bubble disposed within the lumen. The operations may further include correlating one or more impedance measurements with (i) a size of the air bubble, (ii) a location of the air bubble along the medical device, and/or a displacement of the air bubble along the medical device.

Two or more the plurality of electrodes may be disposed on opposite sides of a cross section of the medical device and the electrodes may extend only partially around the cross section.

These and other features of the concepts provided herein will become more apparent to those of skill in the art in view of the accompanying drawings and the following description, which describe particular embodiments of such concepts in greater detail.

### BRIEF DESCRIPTION OF DRAWINGS

A more particular description of the present disclosure will be rendered by reference to specific embodiments thereof that are illustrated in the appended drawings. It is appreciated that these drawings depict only typical embodiments of the invention and are therefore not to be considered limiting of its scope. Example embodiments of the invention will be described and explained with additional specificity and detail through the use of the accompanying drawings in which:
FIG. 1A illustrates a system for monitoring a catheter related process, in accordance with some embodiments;
FIGS. 1B and 1C illustrate exemplary use cases of the system of FIG. 1A, in accordance with some embodiments;
FIG. 2 is a block diagram of a console of the system of FIG. 1A, in accordance with some embodiments;
FIG. 3 illustrates an exemplary embodiment of a monitoring catheter for employment with the system of FIG. 1A, in accordance with some embodiments;
FIG. 4 illustrates an exemplary embodiment of a drainage tube for employment with the system of FIG. 1A, in accordance with some embodiments;
FIG. 5 illustrates an exemplary embodiment of an elongate member configured for insertion within a catheter during employment with the system of FIG. 1A, in accordance with some embodiments; and
FIGS. 6A and 6B illustrate alternative use cases of a second embodiment of a catheter for employment with the system of FIG. 1A, in accordance with some embodiments.

### DETAILED DESCRIPTION

Before some particular embodiments are disclosed in greater detail, it should be understood that the particular embodiments disclosed herein do not limit the scope of the concepts provided herein. It should also be understood that a particular embodiment disclosed herein can have features that can be readily separated from the particular embodiment and optionally combined with or substituted for features of any of a number of other embodiments disclosed herein.

Regarding terms used herein, it should also be understood the terms are for the purpose of describing some particular embodiments, and the terms do not limit the scope of the concepts provided herein. Ordinal numbers (e.g., first, second, third, etc.) are generally used to distinguish or identify different features or steps in a group of features or steps, and do not supply a serial or numerical limitation. For example, "first," "second," and "third" features or steps need not necessarily appear in that order, and the particular embodiments including such features or steps need not necessarily be limited to the three features or steps. Labels such as "left," "right," "top," "bottom," "front," "back," and the like are used for convenience and are not intended to imply, for example, any particular fixed location, orientation, or direction. Instead, such labels are used to reflect, for example, relative location, orientation, or directions. Singular forms of "a," "an," and "the" include plural references unless the context clearly dictates otherwise. The words "including," "has," and "having," as used herein, including the claims, shall have the same meaning as the word "comprising." Furthermore, the terms "or" and "and/or" as used herein are to be interpreted as inclusive or meaning any one or any combination. As an example, "A, B or C" or "A, B and/or C" mean "any of the following: A; B; C; A and B; A and C; B and C; A, B and C." An exception to this definition will occur only when a combination of elements, components, functions, steps or acts are in some way inherently mutually exclusive.

The phrases "connected to" and "coupled to" refer to any form of interaction between two or more entities, including mechanical, electrical, magnetic, electromagnetic, fluid, signal, communicative (including wireless), and thermal interaction. Two components may be connected or coupled to each other even though they are not in direct contact with each other. For example, two components may be coupled to each other through an intermediate component.

Any methods disclosed herein include one or more steps or actions for performing the described method. The method steps and/or actions may be interchanged with one another. In other words, unless a specific order of steps or actions is required for proper operation of the embodiment, the order and/or use of specific steps and/or actions may be modified. Moreover, sub-routines or only a portion of a method described herein may be a separate method within the scope of this disclosure. Stated otherwise, some methods may include only a portion of the steps described in a more detailed method. Additionally, all embodiments disclosed herein are combinable and/or interchangeable unless stated otherwise or such combination or interchange would be contrary to the stated operability of either embodiment.

The directional terms "proximal" and "distal" are used herein to refer to opposite locations on a medical device. The proximal end of the device is defined as the end of the device closest to the end-user when the device is in use by the end-user. The distal end is the end opposite the proximal end, along the longitudinal direction of the device, or the end furthest from the end-user.

Unless defined otherwise, all technical and scientific terms used herein have the same meaning as commonly understood by those of ordinary skill in the art.

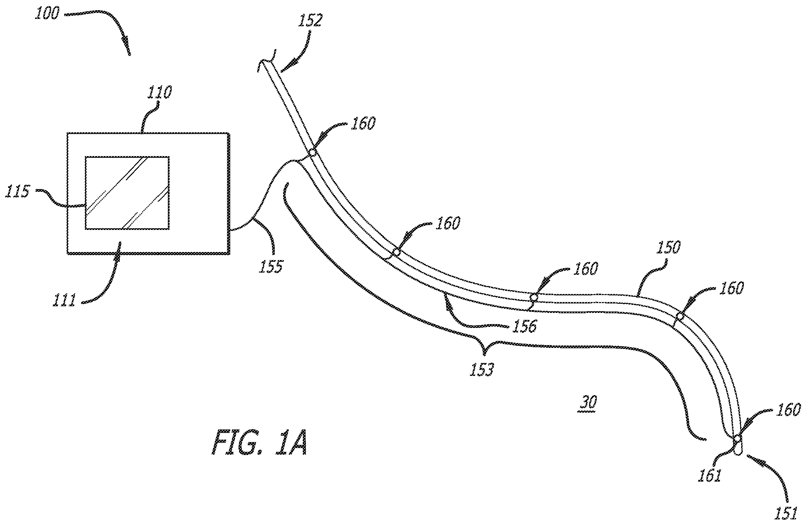

FIG. 1A illustrates an exemplary embodiment of a monitoring system 100. The system 100 generally includes an elongate member 150 electrically coupled with a monitoring module 110 via an electrical cable 155. The elongate member 150 is configured for insertion within a patient body. A plurality of electrodes 160 are disposed along a monitoring length 153 of the elongate member 150 and the electrodes 160 are each electrically coupled with the module 110. In use, the electrodes 160 may be electrically coupled with a substance 30 in contact with the elongate member 150.

The elongate member 150 may include various shapes and structures. In some embodiments, the elongate member 150 may be flexible such as guidewire or a catheter. In other embodiments, the elongate member 150 may be stiff, such as a probe or a needle. The elongate member 150 may be configured for insertion along a vasculature of the patient. In some implementations, the elongate member 150 may be inserted within a lumen of a catheter such as an intravenous catheter or a drainage catheter, for example. The elongate member 150 may define a solid or hollow cross section. In some embodiments, the elongate member may be a catheter, a needle, a stylet, a guidewire, an introducer, or any combination thereof.

The elongate member 150 includes a plurality of conductors 156 (e.g., wires) extending along its length. More specifically, the elongate member 150 includes at least one conductor 156 extending between each electrode 160 and the proximal end 152 and each conductor is electrically coupled with the module 110 via the cable 155. The electrodes 160 may be evenly spaced along the monitoring length 153 or the distances between adjacent electrodes 160 may vary.

Each electrode 160 may be configured to emit or receive electrical signals. One or more electrodes 160 may be configured to both emit and receive electrical signals. Each electrode 160 may be configured to receive an electrical signal in response to an emitted electrical signal by another electrode. A single electrode 160 may receive electrical signals emitted by multiple electrodes 160, and multiple electrodes 160 may receive an electrical signal emitted by a single electrode 160. Similarly, multiple electrodes 160 may receive electrical signals emitted by multiple electrodes 160. In some embodiments, one or more electrodes 160 may be configured to receive an electrical signal emitted by a source other than another electrode 160. For example, the electrode 161 may be configured to receive an EKG signal at a distal end 151 of the elongate member 150. Other sources may include an electrode placed on the skin of the patient.

Each electrode 160 may be selectively configured as an emitting electrode or a receiving electrode. The configuration of one or more electrodes 160 may be defined by the module 110. For example, a specific electrode 160 may be configured to receive electrical signals according to one monitoring mode and the same electrode 160 may be configured to emit electrical signals according to another monitoring mode.

The electrodes 160 may define various physical characteristics. In some embodiments, an electrode 160 may define a two-dimensional shape, such as a circular or rectangular patch, for example. The patch may be disposed on a lateral side of the elongate member and extend only partially (e.g., about 10 to 25 percent) around a circumference of the cross-section of the elongate member 150. In some embodiments, a complementary pair of electrodes 160 may be disposed across from each other on opposite sides of the cross section. Multiple electrodes 160 may also be disposed linearly along a single side of the cross section.

One or more electrodes 160 may also define a cylindrical band shape extending entirely around a circumference of the cross section. The elongate member 150 may include electrodes 160 extending along an outside circumferential surface of the cross section and/or along an inside circumferential surface of a hollow cross section.

The monitoring module 110 is generally configured to cause electrical signals to be emitted from one or more electrodes 160 and receive electrical signals from one or more electrodes 160. The monitoring module 110 is further generally configured to process electrical signals in accordance with operating modes of the system 100. The module 110 generally includes a console 111, and the module 110 may include or be coupled with a display 115. The display 115 may define a graphical user interface (GUI).

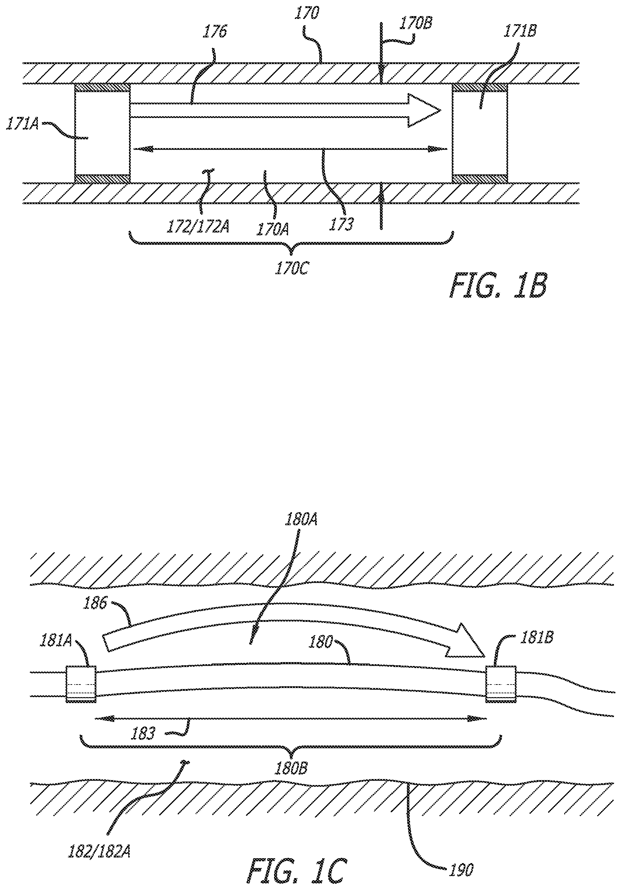

FIG. 1B is an illustration of a general use case the system 100 including a tubular elongate member 170, which may be a subsection of the elongate member 150 of FIG. 1A and/or an alternative embodiment thereof that may in some respects resemble the features and functionality of the elongate member 150 of FIG. 1A. Electrodes 171A, 171B are disposed inside a lumen 170A having a diameter 170B. The electrodes 171A, 171B are spaced apart by a distance 170C. A substance 172 which may be one embodiment of the substance 30 of FIG. 1A, fills the lumen 170A and extends between the electrodes 171A, 171B. The substance 172 may be a fluid having impedance characteristics 172A. Example fluids may include urine, a drainage fluid, saline, a drug, a drug/saline combination or any other fluid that may be present within a catheter. An electrical signal 176 emitted by electrode 171A, passes through the substance 172 to the electrode 171B.

The dimensions of the substance 172 extending between the electrodes 171A, 171B in combination with impedance characteristics 172A of the substance 172, define an electrical impedance 173 between the electrodes 171A, 171B. The impedance 173 is generally proportional to the distance 170C and is generally inversely proportional to a cross-sectional area defined by the diameter 170B. The impedance 176 is related directly to the impedance characteristics 172A. For example, the impedance 176 is inversely proportional to a conductivity of the substance 172. The impedance 176 is measured by the module 110 by passing the electrical signal 176 through the substance 172 between the electrodes 171A, 171B.

A variation in the impedance characteristics 172A of the substance 172 may cause the impedance 173 to vary. For example, a variance in a composition of the substance 172 may define a variance in the impedance characteristics 172A of the substance 172 resulting in a variance in the impedance 173. Therefore, a measured impedance 173 may correlate with a composition of the substance 172. In some instances, the composition of the substance 172 may be defined by a combination of components of the substance 172. For example, in the case of an intravenous catheter, the substance 172 may include a drug component and a saline component having different impedance characteristics. A such, the measured impedance 173 may correlate with a concentration of the drug/saline combination.

Variations in the dimensions of the substance 172 may also cause the impedance 173 to vary. For example, an occlusion of the lumen 170A may decrease the cross-sectional area of the substance 172 causing an increase in the impedance 173. Therefore, a measured impedance 173 may correlate with an occlusion of the lumen 170A. In a similar case, an air bubble present within the lumen 170A may effectively decrease the cross-sectional area of the substance 172 causing an increase in the impedance 173. Therefore, a measured impedance 173 may correlate with the presence of the air bubble.

FIG. 1C is an illustration of another general use case the system 100 including an elongate member 180, which may be a subsection of the elongate member 150 of FIG. 1A and/or an alternative embodiment thereof that may in some respects resemble the features and functionality of the elongate member 150 of FIG. 1A. Electrodes 181A, 181B are disposed on an outside surface 180A of the elongate member 180. The electrodes 181A, 181B are spaced apart by a distance 180B. A substance 182 which may be one embodiment of the substance 30 of FIG. 1A, has impedance characteristics 182A. The substance 182 extends along the outside surface 180A of the elongate member 180 between the electrodes 181A, 181B. An electrical signal 186 emitted by electrode 181A, passes through the substance 182 to the electrode 181B. In the illustrated case, the substance 182 is disposed within a conduit 190 such as a blood vessel. In other cases, the substance 182 may simply surround the elongate member 180, such as body tissue adjacent an elongate member in the form of a probe, for example.

The dimensions of the substance 182 extending between the electrodes 181A, 181B in combination with the impedance characteristics 182A of the substance 182, defines an electrical impedance 183 between the electrodes 181A, 181B. The impedance 183 is generally proportional to the distance 180B and may be generally inversely proportional to a cross-sectional area defined by the conduit 190. The impedance 186 is directly related to the impedance characteristics 182A. For example, in the instant case, impedance 186 may be inversely proportional to a conductivity of the substance 182 (e.g., blood flowing through the conduit 190). The impedance 186 is measured by the module 110 by passing an electrical signal 186 through the substance 182 between the electrodes 181A, 181B.

A variation in the impedance characteristics 182A of the substance 182 may cause the impedance 183 to vary. For example, a variance in a composition of the substance 182 (e.g., blood) may define a variance in the impedance characteristics 182A resulting a variance of the impedance 183. Therefore, a measured impedance 183 may correlate with a composition of the substance 182. In some instances, the composition of the substance 182 may be defined by a combination of components of the substance 182. For example, in the instant case, the blood includes a concentration of red blood cells. As such, the measured impedance 183 may correlate with the concentration of red blood cells (i.e., the hematocrit level of the blood).

Variations in the dimensions of the substance 182 may also cause the impedance 183 to vary. For example, an occlusion of the conduit 190 may decrease the cross-sectional area of the substance 182 causing an increase in the impedance 183. Therefore, a measured impedance 183 may correlate with an occlusion of the conduit 190 (blood vessel), such as a blood clot, for example. In a similar case, an air embolism present within the blood vessel may effectively decrease the cross-sectional area of the substance 182 causing an increase in the impedance 183. Therefore, a measured impedance 183 may correlate with the air embolism.

The use cases of FIGS. 1B, 1C represent only two exemplary cases of many more that could be shown and described. As such, any and all other use cases, as may be contemplated by one of ordinary skill, are disclosed herein.

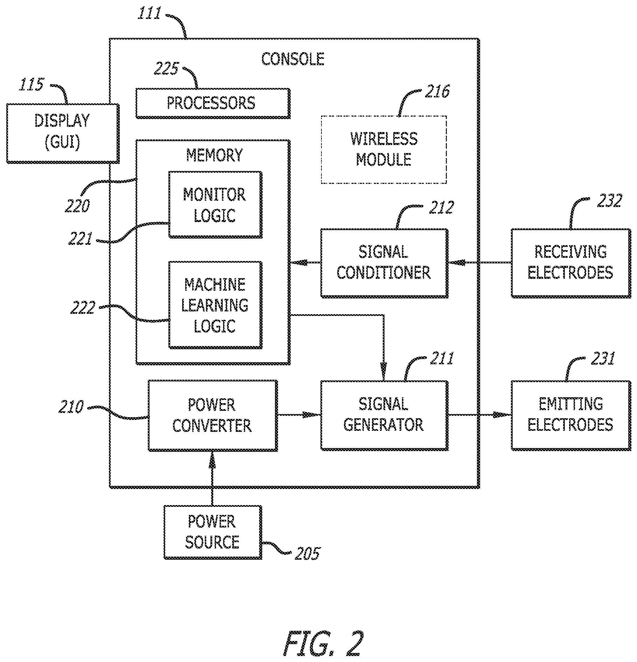

FIG. 2 is a block diagram of the console 111 including modules of the console 111. A power source 205 provides electrical power to the power converter 211 which distributes electrical power to the other modules of the console 111. Monitor logic 221, stored in memory 220 including a non-transitory computer-readable storage medium, defines operations performed by one or more processors 225. A signal generator 211 transmits electrical signals as defined by the monitor logic 221 to the emitting electrodes 231, wherein the emitting electrodes 231 are a subset of the electrodes 160 (see FIG. 1A) defined by the monitor logic 221. A signal conditioner 212 receives electrical signals from the receiving electrodes 232, wherein the receiving electrodes 222 are another subset of the electrodes 160 defined by the monitor logic 221. The signal conditioner 212 may include an analog to digital converter and any other signal conditioning components for converting electrical signals to digital data suitable for processing by the monitor logic 221. Processing results may be rendered on the display 115. In some embodiments, the console 111 may include a wireless module 216 to facilitate data transfer with external computing devices (not shown).

The system 100 may be generally configured to provide information regarding one or more conditions of a local environment including the substance 30 (see FIG. 1A) in contact with and/or extending between two or more electrodes. In some implementations, the substance 30 may be a body substance, such as a body fluid, tissue, or bone, for example. In other implementations, the substance 30 may be associated with a medical treatment, such as a medication. The substance 30 may be disposed within the patient body or external to the patient body. The conditions may include static conditions of the substance 30, such as a hydration state, infiltration of a foreign substance, swelling, composition, etc. The conditions may also include dynamic conditions, such as a motion of substance 30, a pressure pulse, and the like. Monitoring operations defined by the monitor logic 221 and executed by the one or more processors 225 include the processing of received electrical signals to determine one or more conditions of the substance 30.

In use, the system 100 may generally determine electrical properties of the substance 30 surrounding and in contact with the elongate member 150. The logic 221 may process one or more electrical properties of the substance to determine one or more conditions of the substance 30. As discussed above, the conditions may include static conditions such as a composition of the substance 30, and/or dynamic conditions such as a motion of the substance 30.

By way of a general example, the logic 221 may receive electrical signal data from an electrode 160, where the electrical signal data is defined by an electrical property of the substance 30 adjacent the electrode 160. The logic 221 may then process the electrical data via an algorithm stored in memory, where the algorithm correlates electrical properties of the substance 30 with known conditions of the substance 30.

In some implementations, the logic 221 may compare a measured impedance with a predefined impedance range, where the impedance range relates to an expected condition of the substance 30. If the measured impedance is outside the predefined range, the logic 221 may provide a notification to the operator, which notification may be visual or audible. In some implementations, the logic 221 may compare multiple measured impedances with respective predefined impedance ranges, where the impedance ranges relate to multiple expected conditions of the substance 30 such as a composition of the substance 30 at different locations along the elongate member 150 (see FIG. 1A). If any one measured impedance is outside its respective predefined range, the logic 221 may provide a notification to the operator accordingly.

In some embodiments, the system 100 may utilize machine learning techniques (or other artificial intelligence techniques) to enhance system operation, e.g., correlation algorithms of the logic 221. For example, a machine learning logic 222 may be trained utilizing measured impedances under known operating conditions. By way of specific example, a known drug/saline combination flowing through a known elongate member 150 may define impedances as measured along the elongate member catheter 150. The known operating conditions such as flow rate, drug type, and drug concentration, for example, may be manually input to define a training data set. The training data set may then be used to adjust expected impedance ranges to be employed with the logic 221.

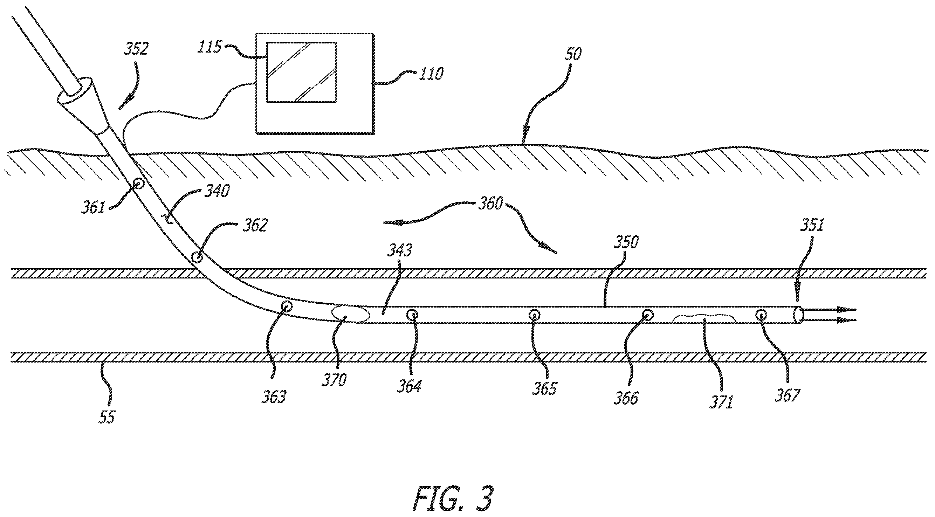

FIG. 3 illustrates an exemplary embodiment of a monitoring catheter 350 coupled with the monitoring module 110. The catheter 350 includes a plurality of electrodes 360 disposed along the catheter 350. By way of example, the electrodes 360 include electrodes 361-367. The electrodes 361-367 are disposed on an inside surface of the catheter 350. In an exemplary implementation, the catheter 350 is inserted within a vasculature 55 of a patient 50 delivering a fluid 340 to the patient 50. Additionally, the monitoring logic 221 causes an operation of the module 110 to include an electrical impedance measurement between any two adjacent electrodes 360.

In a first exemplary instance, an air bubble 370 is disposed within the catheter 350 between electrodes 363, 364. The presence of the bubble 370 reduces the conductance of the fluid portion 343 between electrodes 363, 364 to cause the impedance between electrodes 363, 364 to be greater than the impedance between electrodes 362, 363 and between electrodes 364, 365. As such, the logic 221 may determine the presence of the air bubble 370 and the location of the bubble 370 along the catheter 350. The logic 221 may also track a distal migration of the bubble 370 along the catheter 350. The logic 221 may also render information on the display 115 in accordance with the determination of the air bubble 370. In some implementations, the logic 221 may also determine a size of the bubble 370.

In a second exemplary instance, an occlusion 371 is present within the catheter 350 between electrodes 366, 367. The occlusion 371 may be partially or fully occlude the catheter 350. Similar to the air bubble 370, the occlusion 371 causes the impedance between electrodes 366, 367 to be greater than the impedance between electrodes 365, 366. As such, the logic 221 may determine the presence and location of the occlusion 371 within the catheter 350, and render information on the display 115 accordingly. The occlusion may be a mechanical occlusion such as a kink or crush of the catheter 350. The occlusion may also be a thrombotic or chemical occlusion.

In some implementations, the logic 221 may also determine a magnitude of the occlusion 370. For example, a minimal occlusion may cause only a minimal increase in the impedance between electrodes 366, 367 and occlusion of a higher magnitude may cause a greater increase in the impedance between electrodes 366, 367.

In some implementations, the logic 221 may also determine a flow rate of an infusate through the catheter 350. For example, the logic 221 may track the distal displacement of an interface between a first infusate and a second infusate along the catheter 350. The first infusate may be saline having a first impedance characteristic and the second infusate may be a combination of a drug and the saline having a second impedance characteristic. In use, a flow of saline may be initially established through the catheter 350 followed by a flow of the drug/saline combination. As such, the shift from the first impedance characteristic to the second impedance characteristic distally progresses along the catheter 350 so that the impedance measured between adjacent electrode pairs changes as the shift from the first impedance characteristic to the second impedance characteristic progresses from the 361/362 electrode pair to the 366/367 electrode pair. The logic 221 may correlate the progressive shifting of the measured impedance between adjacent electrode pairs to a flow rate of the infusate.

In some implantations, the monitoring catheter 350 may facilitate catheter placement by monitoring the position of the catheter tip 351 during and/or after placement of the catheter 350. In some implementations, the monitoring catheter 350 may be used to provide a saline column for electrocardiogram (ECG) monitoring related to a position a catheter tip 351 within a superior vena cava. In the recent past, central venous catheters filled with saline have been used to provide an electrically conductive path in leu of a separate electrical conductor to monitor the ECG signal during placement of the catheter tip 351 within a superior vena cava. In such an application, it is advantageous to minimize the electrical impedance along the catheter 350. As such, the logic 221 may monitor the impedance between the electrodes 361-367 to ensure there are no air bubbles 370 or occlusions 371 along the catheter 350 and thereby ensure ECG signal clarity.

In some implementations, the one or more conductors (not shown but see conductors 156 of FIG. 1A) extending between the electrodes 361-367 a proximal end 352 of the catheter 350 may provide the electrically conductive path for ECG monitoring. In some embodiments, the monitoring module 110 may be configured to receive an ECG signal via one or more of the electrodes 360 and the logic 221 may be configured to correlate the ECG signal with a position of the catheter tip 351 with the superior vena cava. The logic 221 may further be configured to provide a notification to the operator when the catheter tip 351 is outside of a predefined position range for the catheter tip 351 within the superior vena cava.

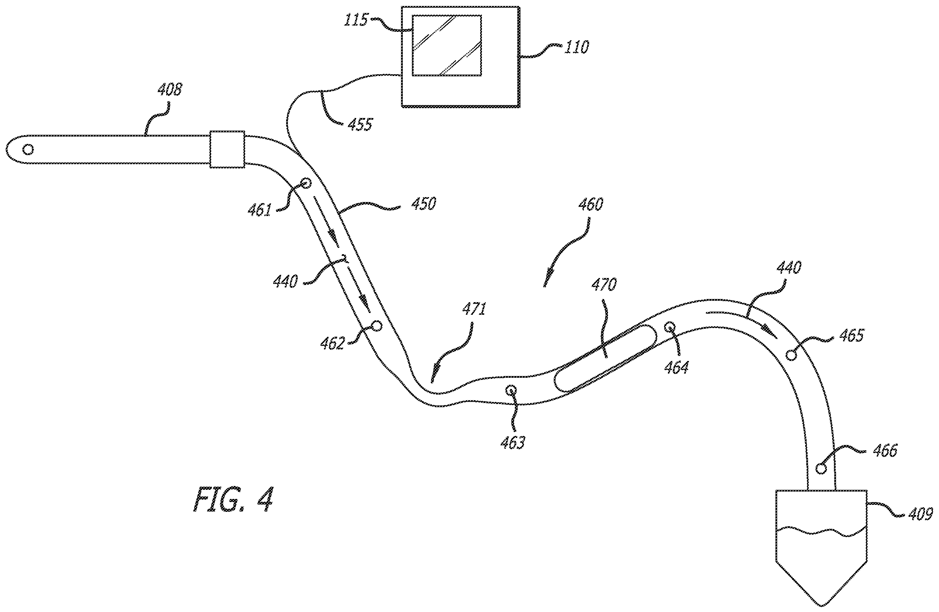

FIG. 4 illustrates an exemplary embodiment of a monitoring drainage tube 450 coupled with the monitoring module 110 via the cable 455. The drainage tube 450 includes a plurality of electrodes 460 disposed along the drainage tube 450. By way of example, the electrodes 460 may include electrodes 461-466. The electrodes 461-466 are disposed on an inside surface of the drainage tube 450. A drainage fluid 440 (e.g., urine) flows through the drainage tube 450 between a drainage catheter 408 and collection container 409. The monitoring logic 221 causes an operation of the module 110 to include electrical impedance measurements between the electrodes 461-466 including any two adjacent electrodes.

In a first exemplary instance, an air pocket 470 is present within the drainage tube 450 between electrodes 463, 464. The presence of the air pocket 470 defines an impedance between adjacent electrodes 463, 464 that is greater than the impedance between adjacent electrodes 464, 465 and electrodes 465, 466. As such, the logic 221 may determine the presence of the air pocket 470 and the location of the air pocket 470 along the drainage tube 450. The logic 221 may also track a migration of the air pocket 470 along the drainage tube 450. The logic 221 may also render information on the display 115 in accordance with presence and condition of the air pocket 470.

In a second exemplary instance, an occlusion 471, which may be a partial occlusion or complete occlusion, is present within the drainage tube 450 between electrodes 462, 463. Similar to the air pocket 470, the partial occlusion 471 causes the impedance between electrodes 462, 463 to be greater than the impedance between electrodes 461, 462. As such, the logic 221 may determine the presence and location of the partial occlusion 471 along the drainage tube 450, and render information on the display 115 accordingly.

In some implementations, the logic 221 may also determine a discontinuation of the fluid flow through the drainage tube 450 such as may be caused by a closure of a drainage cavity within the patient body. In instances of flow stoppage, air pockets may form along the drainage tube 450 particularly adjacent a distal end of the drainage tube 450, thereby causing an increase in the impedance between electrodes 465, 466, for example. In such instances, the logic 221 may correlate the increased impedance between electrodes 465, 466 with a discontinuance of flow of the fluid 440 along the drainage tube 450.

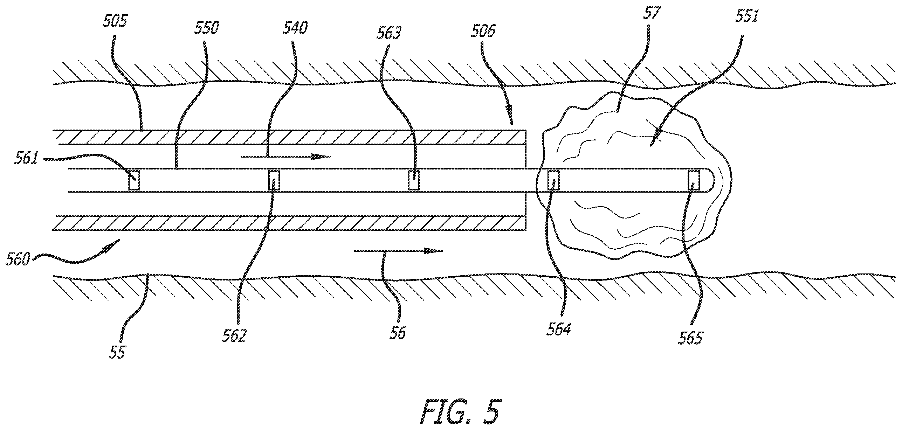

FIG. 5 illustrates an exemplary implementation an elongate member 550, which may be a subsection of the elongate member 150 of FIG. 1A and/or an alternative embodiment thereof may be a guidewire type device for employment with the monitoring module 110 (see FIG. 1A). The elongate member 550 extends through an intravenous catheter 505 having an infusate 540 flowing therethrough. The catheter 505 is disposed with a blood vessel 55 to dispense the infusate 540 into the flow of blood 56. A distal portion 551 of the elongate member 550 extends beyond a distal end 506 of the catheter 505. The elongate member 550 includes a plurality of electrodes 560 disposed along a length the elongate member 550. By way of example, the electrodes 560 may include electrodes 561-565. The electrodes 561-565 are disposed on an outside surface of the elongate member 550 and are coupled with the monitoring module 110. The monitoring logic 221 (FIG. 2) causes an operation of the module 110 to include electrical impedance measurements between the electrodes 561-565 including any two adjacent electrodes.

The electrodes 561-563 are disposed within the catheter 505 and the electrodes 564, 565 are disposed beyond the distal end 506 of the catheter 505. In the illustrated implementation, the fluid volume 57 located adjacent the distal end 506 may composed of infusate 540, blood 56 or a combination of infusate 540 and blood 56. In an instance of flow of infusate 540 through the catheter 505, the fluid volume 57 may be composed of a combination of infusate 540 and blood 56. Conversely, in an instance of no flow of infusate 540 through the catheter 505, the fluid volume 57 may be composed almost solely of blood 56.

Impedance measurements between adjacent electrode pairs may relate to impedance characteristics of the fluid adjacent/surrounding the respective electrode pairs. More specifically, impedance measurements between electrodes 561, 562 and 562, 563 may relate to the impedance characteristics of the infusate 540 and an impedance measurement between electrodes 564, 565 may relate to the impedance characteristics of the fluid volume 57 surrounding the distal portion 551. As such, the impedance measurement between electrodes 564, 565 may relate to a flow rate of infusate 540 exiting the catheter 505.

The logic 221 may correlate the impedance measurement between electrodes 564, 565 with a flow rate of the infusate 540. In an exemplary implementation, the logic 221 may obtain a first impedance measurement between electrodes 564, 565 before initiating the flow of infusate 540 so that the first impedance measurement relates to the impedance characteristics of the blood 56. After initiating the flow of infusate 540, the logic 221 may obtain a second impedance measurement between electrodes 561, 562 so that the second impedance measurement relates to the impedance characteristics of the infusate 540. During infusion of the infusate 540, the logic 221 may monitor the impedance measurement between electrodes 564, 565.

If the monitored impedance migrates toward the first impedance measurement (relating to the blood 56), the logic 221 may correlate the migration with a decrease in the flow rate of the infusate 540 (i.e., a decrease in patency of the catheter 505) as may be caused by an occlusion of the catheter 505. If the monitored impedance migrates toward the second impedance measurement (relating to the infusate 540), the logic 221 may correlate the migration with a decrease in the flow rate of blood 56 through the vessel 55 as may be caused by blood clot. In either scenario, the logic 221 may generate a notification to the clinician/operator, such as information rendered on the display 115 (see FIG. 1A) and/or audible alert.

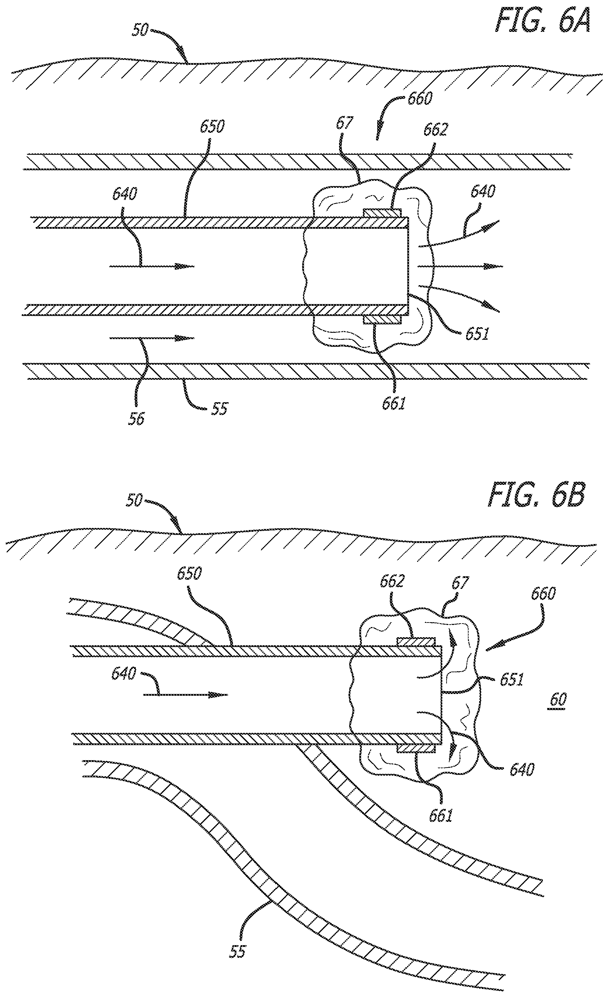

FIGS. 6A and 6B illustrate a monitoring catheter 650 for employment with the monitoring module 110 (see FIG. 1A) in two alternative use scenarios. FIG. 6A illustrates a catheter tip 651 disposed properly within the blood vessel 55 having blood 56 flowing therethrough, and FIG. 6B illustrates a catheter tip 651 disposed improperly within body tissue 60 outside the blood vessel 55. The catheter 660 includes a plurality of electrodes 660 including electrodes 661, 662 coupled with the monitoring module 110 (see FIG. 1A). The electrodes 661, 662 are disposed on an outside surface of the catheter 660 adjacent the catheter tip 651 so as to be facing away from each other on opposite sides of the catheter 660. The monitoring logic 221 causes an operation of the module 110 to include electrical impedance measurements between the electrodes 661, 662. The electrodes 661, 662 are disposed within a fluid volume 67 extending proximally away from the distal tip 651 so that an impedance measurement between the electrodes 661, 662 is related to impedance characteristics of the fluid volume 67.

In FIG. 6A, infusate 640, flowing through the catheter 660, exits the catheter 660 at the distal tip 651 to join the blood 56 flowing distally away from the distal tip 651. As such, the fluid volume 67 is composed solely of blood 56 and therefore, an impedance measurement between electrodes 661, 662 relates to the impedance characteristics of the blood 56. By way of contrast in FIG. 6B, infusate 640, flowing through the catheter 660, exits the catheter 660 at the distal tip 651 into the into body tissue 60 via infiltration. As such, infusate 640 flows into the fluid volume 67. As the fluid volume 67 includes infusate 640, an impedance measurement between electrodes 661, 662 relates at least partially to the impedance characteristics of the infusate 640.

The logic 221 may correlate the impedance measurement between electrodes 661, 662 with the blood 56 or the infusate 640. In an event where the correlation indicates an impedance related to the blood 56, the logic 221 may determine that the distal tip 651 is disposed within the blood vessel 55. In an alternative event where the correlation indicates an impedance related to the infusate 640, the logic 221 may determine that the distal tip 651 is improperly positioned outside the blood vessel 55. A correlation indicating an impedance related to infusate 640 indicates an infiltration of the infusate 640. In either scenario, the logic 221 may generate a notification to the clinician, such as information rendered on the display 115 (see FIG. 1) pertaining to the location of the distal tip 651.

Without further elaboration, it is believed that one skilled in the art can use the preceding description to utilize the invention to its fullest extent. The embodiments disclosed herein are to be construed as merely illustrative and exemplary, and not a limitation of the scope of the claims. It will be apparent to those having ordinary skill in the art, with the aid of the present disclosure, that changes may be made to the details of the above-described embodiments without departing from the scope of the claims. In other words, various modifications and improvements of the embodiments specifically disclosed in the description above are within the scope of the appended claims. Moreover, the order of the steps or actions of the methods disclosed herein may be changed by those skilled in the art without departing from the scope of the claims. In other words, unless a specific order of steps or actions is required for proper operation of the embodiment, the order or use of specific steps or actions may be modified. The scope of the invention is therefore defined by the following claims.

## Claims

1. A system (100) for monitoring a medical process, comprising:
a medical device (150; 170; 350;450; 550; 650) configured for insertion within a vasculature of a patient;
a plurality of electrodes (160; 171A,B; 360; 460; 560; 660) coupled with the medical device; and
a monitoring module (110) electrically coupled with the plurality of electrodes, the module including logic stored in memory that, when executed by one or more processors (225), causes performance of operations including:
transmitting an electrical signal between a first electrode and a second electrode,
determining an electrical impedance between the first electrode and the second electrode,
correlating one or more impedance measurements with an infiltration of an infusate, and
providing a notification to the operator when the determined electrical impedance is outside a predefined impedance range.

2. The system of claim 1, wherein the medical device is a catheter, a needle, a stylet, a guidewire, an introducer, or a combination thereof, and wherein the electrodes are disposed along a monitoring length of the medical device.

3. The system of either of claims 1 or 2, wherein the operations include:
transmitting electrical signals between a plurality of first electrodes and a plurality of second electrodes,
determining electrical impedances between a plurality of electrode pairs, and
providing a notification to the operator when one or more determined electrical impedances is outside one or more predefined impedance ranges.

4. The system of any of the preceding claims, wherein at least a subset of the plurality of electrodes is disposed within a lumen of the medical device.

5. The system of any of the preceding claims, wherein the operations include correlating one or more impedance measurements with an occlusion of the lumen.

6. The system of claim 5, wherein the occlusion is one of a thrombotic occlusion, a chemical occlusion, or a mechanical occlusion.

7. The system of either of claims 5 or 6, wherein the operations include correlating one or more impedance measurements with a magnitude of the occlusion.

8. The system of any of claims 5-7, wherein the operations include correlating one or more impedance measurements with a location of the occlusion along the medical device.

9. The system of any of the preceding claims, wherein the operations include correlating one or more impedance measurements with an air bubble disposed within the lumen.

10. The system of claim 9, wherein the operations include correlating one or more impedance measurements with a size of the air bubble.

11. The system of either of claims 9 or 10, wherein the operations include correlating one or more impedance measurements with a location of the air bubble along the medical device.

12. The system of any of claims 9-11, wherein the operations include correlating one or more impedance measurements with a displacement of the air bubble along the medical device.

13. The system of any of the preceding claims, wherein two or more the plurality of electrodes are disposed on opposite sides of a cross section of the medical device.

14. The system of claim 13, wherein the two or more of the plurality of electrodes extend only partially around the cross section.

## Patentansprüche

1. System (100) zum Überwachen eines medizinischen Prozesses, umfassend:
eine medizinische Vorrichtung (150; 170; 350; 450; 550; 650), die zur Einführung in das Gefäßsystem eines Patienten ausgebildet ist;
eine Vielzahl von Elektroden (160; 171A,B; 360; 460; 560; 660), die mit der medizinischen Vorrichtung gekoppelt sind; und
ein Überwachungsmodul (110), das elektrisch mit der Vielzahl von Elektroden gekoppelt ist, wobei das Modul Logik einschließt, die in einem Speicher gespeichert ist, die, wenn sie von einem oder mehreren Prozessoren (225) ausgeführt wird, die Ausführung von Vorgängen veranlasst, einschließend:
Übertragung eines elektrischen Signals zwischen einer ersten Elektrode und einer zweiten Elektrode,
Bestimmung einer elektrischen Impedanz zwischen der ersten Elektrode und der zweiten Elektrode,
Korrelation einer oder mehrerer Impedanzmessungen mit einer Infiltration eines Infusats, und
Bereitstellung einer Benachrichtigung an den Bediener, wenn die bestimmte elektrische Impedanz außerhalb eines vordefinierten Impedanzbereichs liegt.

2. System nach Anspruch 1, wobei die medizinische Vorrichtung ein Katheter, eine Nadel, ein Stilett, ein Führungsdraht, eine Einführvorrichtung oder eine Kombination davon ist und wobei die Elektroden entlang einer Überwachungslänge der medizinischen Vorrichtung angeordnet sind.

3. System nach Anspruch 1 oder 2, wobei die Vorgänge Folgendes einschließen:
Übertragung elektrischer Signale zwischen einer Vielzahl von ersten Elektroden und einer Vielzahl von zweiten Elektroden,
Bestimmung elektrischer Impedanzen zwischen einer Vielzahl von Elektrodenpaaren, und
Bereitstellung einer Benachrichtigung an den Bediener, wenn eine oder mehrere der bestimmten elektrischen Impedanzen außerhalb eines oder mehrerer vordefinierter Impedanzbereiche liegen.

4. System nach einem der vorstehenden Ansprüche, wobei mindestens eine Teilmenge der Vielzahl von Elektroden innerhalb eines Lumens der medizinischen Vorrichtung angeordnet ist.

5. System nach einem der vorstehenden Ansprüche, wobei die Vorgänge Korrelation einer oder mehrerer Impedanzmessungen mit einer Okklusion des Lumens einschließen.

6. System nach Anspruch 5, wobei die Okklusion eine thrombotische Okklusion, eine chemische Okklusion oder eine mechanische Okklusion ist.

7. System nach Anspruch 5 oder 6, wobei die Vorgänge Korrelation einer oder mehrerer Impedanzmessungen mit dem Ausmaß der Okklusion einschließen.

8. System nach einem der Ansprüche 5-7, wobei die Vorgänge Korrelation einer oder mehrerer Impedanzmessungen mit der Position der Okklusion entlang der medizinischen Vorrichtung einschließen.

9. System nach einem der vorstehenden Ansprüche, wobei die Vorgänge Korrelation einer oder mehrerer Impedanzmessungen mit einer innerhalb des Lumens angeordneten Luftblase einschließen.

10. System nach Anspruch 9, wobei die Vorgänge Korrelation einer oder mehrerer Impedanzmessungen mit einer Größe der Luftblase einschließen.

11. System nach Anspruch 9 oder 10, wobei die Vorgänge Korrelation einer oder mehrerer Impedanzmessungen mit einer Position der Luftblase entlang der medizinischen Vorrichtung einschließen.

12. System nach einem der Ansprüche 9-11, wobei die Vorgänge Korrelation einer oder mehrerer Impedanzmessungen mit einer Verschiebung der Luftblase entlang der medizinischen Vorrichtung einschließen.

13. System nach einem der vorstehenden Ansprüche, wobei zwei oder mehr der Vielzahl von Elektroden auf gegenüberliegenden Seiten eines Querschnitts der medizinischen Vorrichtung angeordnet sind.

14. System nach Anspruch 13, wobei sich die zwei oder mehr der Vielzahl von Elektroden nur teilweise um den Querschnitt herum erstrecken.

## Revendications

1. Système (100) pour surveiller un procédé médical, comprenant :
un dispositif médical (150 ; 170 ; 350 ; 450 ; 550 ; 650) configuré pour une insertion dans le système vasculaire d'un patient ;
une pluralité d'électrodes (160 ; 171A, B ; 360 ; 460 ; 560 ; 660) couplées au dispositif médical ; et
un module de surveillance (110) couplé électriquement à la pluralité d'électrodes, le module incluant une logique stockée en mémoire qui, lorsqu'elle est exécutée par un ou plusieurs processeurs (225), provoque l'exécution d'opérations incluant :
une transmission d'un signal électrique entre une première électrode et une deuxième électrode, une détermination d'une impédance électrique entre la première électrode et la deuxième électrode,
une corrélation d'une ou plusieurs mesures d'impédance avec une infiltration d'un perfusat, et
une fourniture d'une notification à l'opérateur lorsque l'impédance électrique déterminée est en dehors d'une plage d'impédance prédéfinie.

2. Système selon la revendication 1, dans lequel le dispositif médical est un cathéter, une aiguille, un stylet, un fil-guide, un introducteur, ou une combinaison de ceux-ci, et dans lequel les électrodes sont disposées le long d'une longueur de surveillance du dispositif médical.

3. Système selon la revendication 1 ou la revendication 2, dans lequel les opérations incluent : une transmission de signaux électriques entre une pluralité de premières électrodes et une pluralité de deuxièmes électrodes, une détermination d'impédances électriques entre une pluralité de paires d'électrodes, et une fourniture d'une notification à l'opérateur lorsqu'une ou plusieurs impédances électriques déterminées sont en dehors d'une ou plusieurs plages d'impédance prédéfinies.

4. Système selon l'une quelconque des revendications précédentes, dans lequel au moins un sous-ensemble de la pluralité d'électrodes est disposé dans une lumière du dispositif médical.

5. Système selon l'une quelconque des revendications précédentes, dans lequel les opérations incluent une corrélation d'une ou plusieurs mesures d'impédance avec une occlusion de la lumière.

6. Système selon la revendication 5, dans lequel l'occlusion est une occlusion parmi une occlusion thrombotique, une occlusion chimique, ou une occlusion mécanique.

7. Système selon l'une des revendications 5 ou 6, dans lequel les opérations incluent une corrélation d'une ou plusieurs mesures d'impédance avec une ampleur de l'occlusion.

8. Système selon l'une quelconque des revendications 5 à 7, dans lequel les opérations incluent une corrélation d'une ou plusieurs mesures d'impédance avec un emplacement de l'occlusion le long du dispositif médical.

9. Système selon l'une quelconque des revendications précédentes, dans lequel les opérations incluent une corrélation d'une ou plusieurs mesures d'impédance avec une bulle d'air disposée dans la lumière.

10. Système selon la revendication 9, dans lequel les opérations incluent une corrélation d'une ou plusieurs mesures d'impédance avec une taille de la bulle d'air.

11. Système selon l'une des revendications 9 ou 10, dans lequel les opérations incluent une corrélation d'une ou plusieurs mesures d'impédance avec un emplacement de la bulle d'air le long du dispositif médical.

12. Système selon l'une quelconque des revendications 9 à 11, dans lequel les opérations incluent une corrélation d'une ou plusieurs mesures d'impédance avec un déplacement de la bulle d'air le long du dispositif médical.

13. Système selon l'une quelconque des revendications précédentes, dans lequel au moins deux électrodes parmi la pluralité d'électrodes sont disposées sur des côtés opposés d'une section transversale du dispositif médical.

14. Système selon la revendication 13, dans lequel les au moins deux électrodes parmi la pluralité d'électrodes ne s'étendent que partiellement autour de la section transversale.
